(19) Europäisches Patentamt
European Patent Office
Office européen des brevets

(11) Publication number: **0 171 277**
**A2**

# (12) EUROPEAN PATENT APPLICATION

(21) Application number: **85305537.4**

(22) Date of filing: **02.08.85**

(51) Int. Cl.⁴: **C 07 D 471/04**, C 07 D 513/04, C 07 D 498/04
//
(C07D471/04, 231:00, 221:00),
(C07D471/04, 239:00, 221:00),
(C07D513/04, 277:00, 221:00),
(C07D498/04, 263:00, 221:00)

(30) Priority: **03.08.84 US 637665**
**03.08.84 US 637354**

(43) Date of publication of application: **12.02.86**
**Bulletin 86/7**

(84) Designated Contracting States: **AT BE CH DE FR GB IT LI LU NL SE**

(71) Applicant: **ELI LILLY AND COMPANY, Lilly Corporate Center, Indianapolis Indiana 46285 (US)**

(72) Inventor: **Schaus, John Mehnert, 5427 North Delaware Street, Indianapolis Indiana 46220 (US)**
Inventor: **Whitaker, Nancy Gail Gallick, 9425 Kungsholm Drive Apt.F, Indianapolis Indiana 46250 (US)**

(74) Representative: **Tapping, Kenneth George et al, Erl Wood Manor, Windlesham Surrey, GU20 6PH (GB)**

(54) **Improvements in or relating to tricycling quinoline derivatives.**

(57) A process for preparing trycyclic quinoline derivatives having dopamine agonist properties by reducing the corresponding lactam precursors.

EP 0 171 277 A2

# IMPROVEMENTS IN OR RELATING TO TRICYCLIC QUINOLINE DERIVATIVES

This invention relates to a novel method for preparing tricyclic quinoline derivatives, and to novel intermediates of value in that process.

U.S. Patent Specification No. 4,198,415; J. Med. Chem., 23, 481 (1980) and J.P.E.T., 224, 206 (1982) disclose the dopamine agonist and hypotensive properties of a class of pyrazoloquinoline derivatives which can be prepared by the process of the invention.

According to the present invention there is provided a process for preparing a tricyclic quinoline derivative of formula:

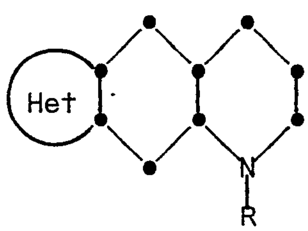

(I)

where the

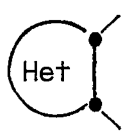

group represents:

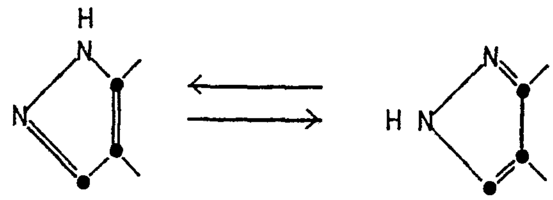

,

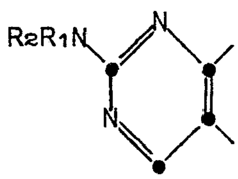

,

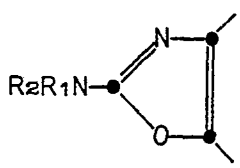

or

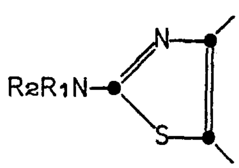

where $R_1$ and $R_2$ are independently hydrogen or $C_{1-3}$ alkyl; and R is hydrogen, $C_{1-3}$ straight chain alkyl or allyl; or a pharmaceutically-acceptable salt thereof; which process comprises reducing a lactam of formula:

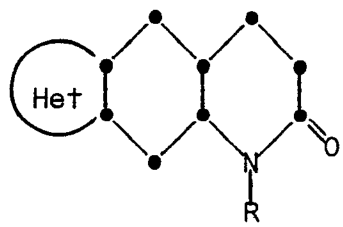

(II)

where the ring-fusion of the non-aromatic rings is <u>trans</u>, and where the heterocyclic ring and R are as above defined.

The reduction should be carried out using a chemical reducing agent such as diborane or lithium aluminum hydride. The reaction can be carried out at temperatures in the range from 0 to 100°C, preferably 20 to 70°C. Typical solvents include aprotic polar organic solvents such as tetrahydrofuran and dimethoxyethane.

The ketones of formula (II) are novel, and are provided in a further aspect of the invention.

The compounds of formula (I) are dopamine agonists.

The pyrazole intermediate of formula II can be prepared by the following reaction schedule. For ease of illustration, although the reaction produces a racemic product, only one enantiomer is depicted:

**Synthetic Route 1**

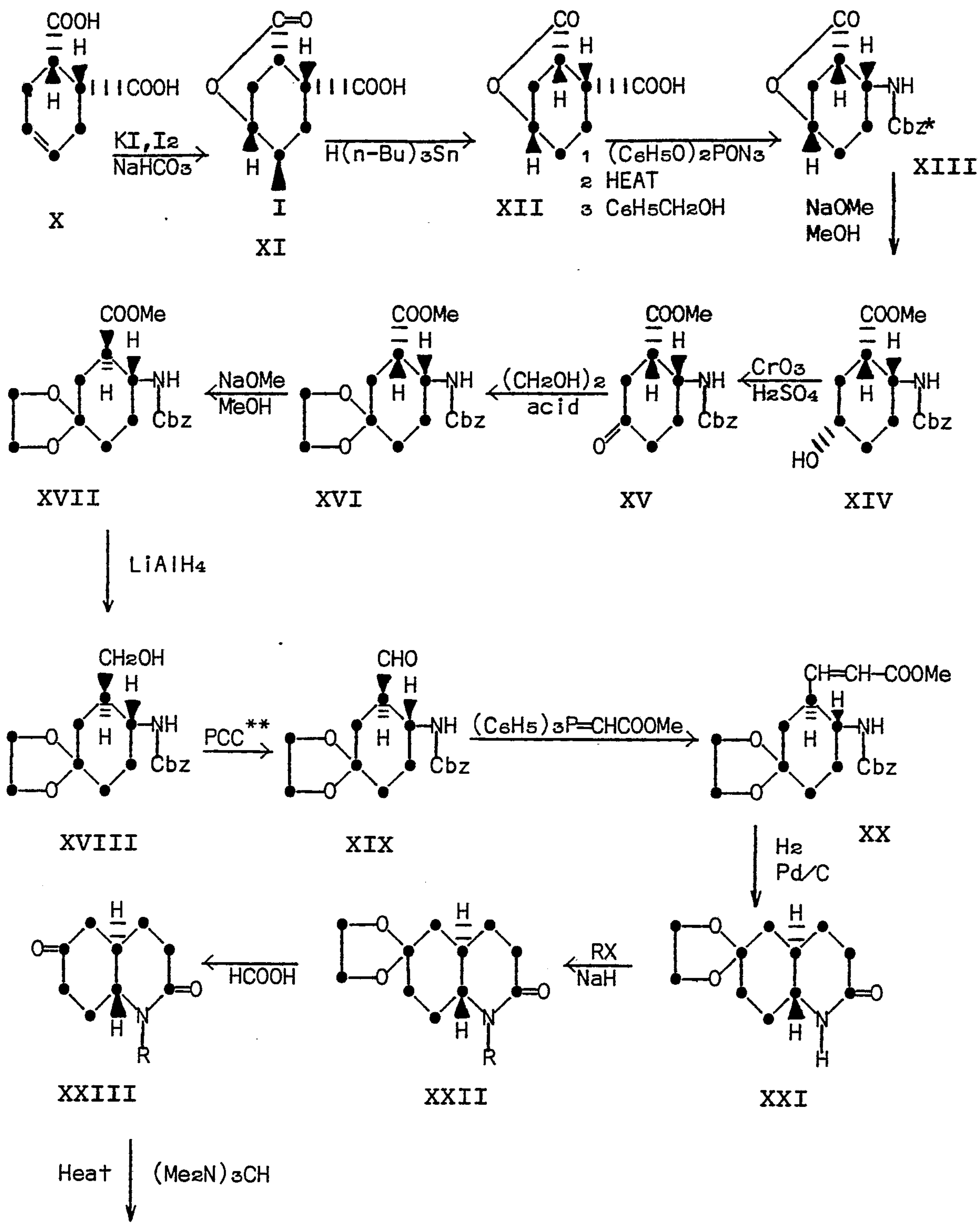


X XI XII XIII

XVII XVI XV XIV

XVIII XIX XX

XXIII XXII XXI

Synthetic Route 1 cont'd.

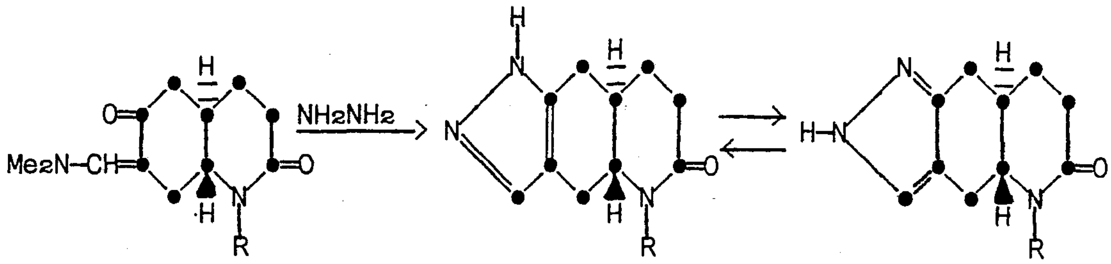


XXIV XXVa XXVb

wherein R is H, $C_{1-3}$ straight-chain alkyl or allyl, *Cbz is CO-O-benzyl; **PCC is pyridinium chlorochromate.

In the above formulas, XIII thru XX, an amine protecting group, Bbz or benzyloxycarbonyl, is illustrated but it will be apparent that other protecting groups of the formula CO-W, wherein W is $OC_{1-3}$ alkyl or O-phenyl $C_{1-2}$ alkyl could be used.

In the above Synthetic Route I, cis-1,2,3,6-tetrahydrophthalic acid (X) is reacted with iodine and KI in sodium bicarbonate solution to yield cis-(±)-5-iodoperhydrophthalic acid 2,4-cyclolactone (XI). Treatment of this derivative with tri-n-butyltin hydride removes the iodo group (XII). Next, a Curtius Rearrangement is carried out on the free carboxylic acid group at C-1 in XII with DDPA (diphenylphosphorylazide). After heating the reaction mixture, benzyl alcohol is added to produce a cis-(±)-6-benzyloxycarbonylamino-perhydrobenzoic acid, 1,3-cyclolactone (XIII). The lactone ring is then opened with base, preferably sodium methylate in methanol, to form methyl cis-(±)-6-benzyloxycarbonylamino-3-hydroxycyclohexanecarboxylate (XIV). Oxidation of the secondary alcohol with Jones Reagent (chromic anhydride in dilute sulfuric acid) yields the corresponding 3-oxo derivative (XV). This ketone group is next protected by ketal formation as by reaction with ethyleneglycol in the presence of acid (XVI). This ketal is next isomerized with base, again preferably with sodium methylate in methanol to avoid transesterification problems, to give methyl trans-(±)-6-benzyloxycarbonylamino-3-ethyleneketalcyclohexylcarboxylate (XVII). The methyl ester group is next reduced to a primary alcohol (XVIII) with lithium aluminum hydride or other reducing agent with similar reducing capability. The primary alcohol is then oxidized to the corresponding aldehyde [XIX] using pyridinium chlorochromate which aldehyde is in turn reacted with the Wittig reagent, methyl (triphenylphosphoranylidene)acetate, to give methyl trans-(±)-3-(2-benzyloxycarbonylamino-5-ethyleneketal)acrylate (XX). Hydrogenation over a noble metal catalyst, conveniently 5% Pd/C, reduces the acrylate olefin and cleaves the carbobenzyloxy protecting group to give an intermediate which spontaneously cyclizes to trans-(±)-2,6-dioxo-6-ethyleneketaldecahydroquinoline (XXI). The ring nitrogen is alkylated or allylated to give a N-$C_{1-3}$ straight-chain alkyl or allyl derivative using conveniently an alkyl iodide or allyl halide and a suitable base such as sodium hydride. Next, the ketal protecting group is removed by acidic treatment, and the resulting trans-(±)-2,6-dioxo-1-substituted-decahydroquinoline reacted with tris-dimethylaminomethane to form the 7-dimethylaminomethylene derivative-(XXIV). Reaction with hydrazine produces trans-(±)-5-$C_{1-3}$ straight-chain alkyl or allyl-6-oxo-4,4a,5,6,7,8,8a,9-octahydro-1H(and 2H)pyrazolo[3,4-g]quinoline (XXVa and XXVb). Reduction of the lactam with lithium aluminum hydride or the like reducing agent yields a pyrazolo derivative of formula (I).

The corresponding tautomeric pair when R is H can prepared from XXI by eliminating the alkylation and carrying out the remaining procedure -- removal of the ketal protecting group with acid, reaction with tris dimethylaminomethane at C-7, ring closure with hydrazine followed by $LiAlH_4$ reduction of the lactam carbonyl to yield ultimately trans-(±)-4,4a,5,6,7,8,8a,9-octahydro-1H(and 2H)-pyrazolo[3,4-g]quinoline, IIIa and IIIb when R is H. This tautomeric pair can be transformed to tautomers bearing a quinoline ring nitrogen substituent; ie., R = methyl,ethyl, n-propyl or allyl, by standard alkylation procedures using a base and an alkyl or allyl halide, care being taken to avoid such stringent reaction conditions that the pyrazole ring is also alkylated.

The above procedure yields a trans-(±)-racemate, which can be resolved using D-(S)-tartaric acid to yield the 4aR,8aR and 4aS,8aS tautomeric pairs. However, the dioxo derivative, XVa and XVb, or the corresponding compound wherein R is H, can also be resolved into its enantiomeric tautomers, 4aR,8aR-6-oxo-4,4a,5,6,7, 8,8a,9-octahydro-1H(and 2H)-pyrazolo[3,4-g]-quinolines and the corresponding 4aS,8aS tautomers.

Likewise, the bicyclic trans-(±)-2,6-dioxo derivatives XXIII can also be resolved into its enantiomers, 4aR,8aR-2,6-dioxo-5-$C_{1-3}$ straight-chain alkyl or allyl-decahydroquinoline and the corresponding 4aS,8aS derivative.

Each enantiomer can then undergo separately the reactions XXIII → XXIV → XXVa + XXVb → IIIa + IIIb; ie., transformation to the 6-oxo-octahydro-pyrazoloquinoline and removal of the oxo group by $LiAlH_4$ reduction to yield where R is n-propyl 4aR,8aR-5-n-propyl-4,4a,5,6,7,8,8a,9-octahydro-1H(and 2H)-pyrazolo[3,4-g]-quinoline or the corresponding 4aS,8aS enantiomer, depending on which enantiomeric 6-oxodecahydroquinoline is used. Thus, this invention provides the racemates XXVa ⇆ XXVb as well as a procedure for preparing both racemates and individual enantiomers.

While the procedure in Synthetic Route I produces only racemic materials, the individual enantiomers, the 6-oxo-octahydro-1H(and 2H)-pyrazolo-[3,4-g]quinolines, IIIa and IIIb, the 6-oxo-octahydro-thiazolo[4,5-g]quinolines, XXXa and XXXb, the 6-oxo-octahydro-oxozolo[4,5-g]quinolines, XXXIIa and XXXIIb, and the 7-oxo-octahydropyrimido[4,5-g]quinolines, XXXVa and XXXVc, can be prepared by resolution of the racemates. Alternatively, the trans-(±)-1-permissibly-substituted-2,6-dioxodecahydroquinoline, XXXVIIa (4aR,8aR) and XXXVIIb (4aS,8aS)

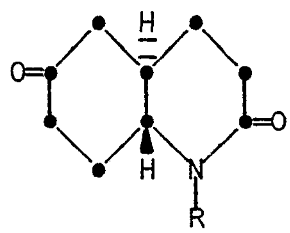

XXXVIIa

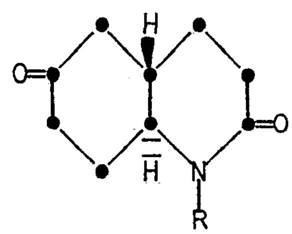

XXXVIIb

can be resolved by ketalization with (+)- or (-)-2,3-butanediol, chromatographic separation of the diastereomers followed by removal of the ketal to yield an optically-active ketone. The enantiomeric secondary amide (R = H is XXXVIIa or XXXVIIb) can then be alkylated or allylated and the resulting tertiary amine cyclized to a pyrazole, thiazole, oxazole, or pyrimidine containing a lactam group and the lactam reduced with $LiAlH_4$ to yield the D-2 or D-1 dopamine agonist directly. Alternatively, the enantiomeric secondary amine (R = H) in XXXVIIa or in XXXVIIb can be annelated, the lactam reduced and the resulting compound allylated or alkylated to yield enantiomeric 4aR,8aR or 5aR,9aR dopamine D-2 agonist or an entantomeric 4aS,8aS or 5aS,9aS dopamine D-1 agonist.

Alternatively, a carboxylic ester XIV, XV, XVI, XVII, or XX from Synthetic Route 1 can be hydrolyzed to the corresponding carboxylic acid and the acid treated with an optically active base to produce diastereomers which can be separated chromatographically or by crystallization. The optically-active esters can then readily be reconstituted by ester cleavage. Also,

compound XVIII is an alcohol which can be esterified with an optically active acid, the resulting diastereomers separated and the optically active alcohol obtained by hydrolysis. Similarly, the protected α-amino esters XIV, XV, XVI, XVII and XVIII can be deprotected, the primary amine neutralized with an optically-active acid, the diastereoisomeric salts separated, the optically-active amine recovered from the salt and the amine group reprotected with the same or a different group.

Again, alternatively, the acids XI or XII can be neutralized by an optically-active amine and the salts separated by recrystallization. Acidification allows for the isolation of the optically-active acids. See Angew. Chem. 2nd Ed. English 23 (1), 67 (1984) for the preparation of cis-(±)-1,2,3,5-tetrahydrophthalic acid, monomethyl ester and separation of the enantiomeric half-acid esters to yield a >94% ee enantiomer.

4aR,8aR-6-oxopyrazole[3,4-g]quinolines of formula II are metabolic products of the corresponding tautomeric 6-dihydro derivatives:

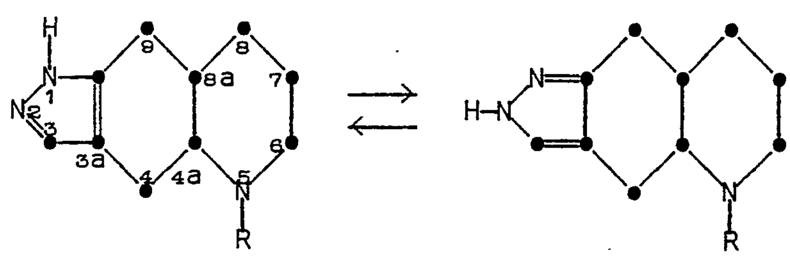

wherein R is hydrogen, $C_{1-3}$ straight-chain alkyl (methyl, ethyl, n-propyl) or allyl, and wherein the stereoconfiguration at 4a and 8a is trans.

In Synthetic Route 1, X is a meso form and not optically active since it has a plane of symmetry. XI since it does not have a plane of symmetry is optically-active; is a cis-(±) or racemic form. However, in XI and all succeeding formulas thru XIX, only one of the cis or trans (as the case may be) enantiomers is pictured in order to simplify the reaction scheme, but a racemate is actually involved. In the bicycles XXI, XXII, XXIII, XXIV, XXVa ⇆ XXVb and IIIa ⇆ IIIb, no orientations are given but only the trans-(±) forms are prepared and thus only these forms are indicated by the structural formulas.

## Example 1

Preparation of Cis-(±)-5-iodoperhydrophthalic acid, 2,4-cyclolactone

A solution was prepared by dissolving 17.0 g. of cis-1,2,3,6-tetrahydrophthalic acid 100 ml. of 10% aqueous sodium bicarbonate. An aqueous solotion of KI and $I_2$ was added thereto. The two solutions were thoroughly mixed and then allowed to stand at ambient temperature for 16 hrs. The reaction mixture was then shaken with saturated aqueous sodium thiosulfate to destroy excess iodine, as shown by the disappearance of a dark-brown color. The alkaline aqueous mixture was extracted with ether, and the ether extract separated and dried. Evaporation of the ether gave a pink residue weighing 22.3 g. The residue was treated with hot ethyl

acetate, and the resulting mixture filtered. The filtrate was diluted with two volumes of hexane. Fine white needles of cis-(±)-5-iodoperhydrophthalic acid, 2,4-cyclolactone precipitated and were collected by filtration; M.P. = 161-164°C. The yield was 61%.

Analysis; Calc.; C, 32.46; H, 3.06; I, 42.86;
Found; C, 32.19; H, 2.89; I, 42.68.
Infrared spectrum; 3190, 1763, 1724 $cm^{1}$
Ultraviolet spectrum; maxima at 207 (ε = 330), 259 (ε = 620).

## Example 2

Preparation of Cis-(±)-perhydrophthalic acid, 2,4-cyclolactone

A solution was prepared by dissolving 60 g. of cis-(±)-5-iodoperhydrophthalic acid, 2,4-cyclolactone in 200 ml. of 1,2-dimethoxyethane. To this solution was added 70.8 g. of tri-n-butyltinhydride. The reaction mixture was kept at room temperature for 3 days and was then poured into 10% aqueous sodium bicarbonate. The aqueous mixture was extracted thoroughly with ether, and the ether extract itself extracted with 10% aqueous sodium bicarbonate. The bicarbonate solutions were combined and then acidified with hydrochloric acid. The now-acidic layer was extracted with a 1:3 isopropanol/-chloroform solvent mixture. The organic extract was evaporated to dryness to give a solid yellow residue

weighing 34.1 g. Recrystallization of the solid from ethyl acetate/hexane gave 18.6 g of 1st crop and 10.4 g. of 2nd crop white crystalline cis-(±)-perhydrophthalic acid, 2,4-cyclolactone.

## Example 3

Preparation of Cis-(±)-2-benzyloxycarbonylaminocyclohexanecarboxylic acid, 1,3-cyclolactone

A solution was prepared by dissolving 18.6 g. of cis-(±)-perhydrophthalic acid, 2,4-cyclolactone in 235 ml. of THF. 12.1 g. of triethyl amine were added thereto. Next, a solution of 31.6 g. of diphenylphosphoryl azide in 50 ml. of THF was added in dropwise fashion at room temperature. The reaction mixture was warmed at about 30°C. for 20 min. and then at reflux temperature for about 3 hrs. The reaction mixture was then cooled to room temperature and stirred overnight at that temperature overnight. 12.4 ml. of benzyl alcohol were added. This reaction mixture was heated to reflux for three hours and was then poured into water. The aqueous mixture was extracted with methylene dichloride. (The pH of the aqueous layer was about 7). The organic extract was dried, and the volatile constituents removed to leave a colorless oil weighing 50.6 g. The residual oil was dissolved in ether, and the ethereal solution chromatographed over silica. Initial fractions were shown to be unreacted benzyl alcohol. The next material to be eluted was cis-(±)-2-benzyloxycarbonylaminocyclohexanecarboxylic acid, 1,3-cyclolactone. 9.5 g. of a white solid product were obtained.

## Example 4

Preparation of Methyl Cis-(±)-2-benzyloxycarbonylamino-5-hydroxycyclohexanecarboxylate

A solution was prepared by dissolving 1.19 g. of Na in 250 ml. of MeOH. A second solution of 14.2 g. of the cyclolactone from Example 3 in 50 ml. of MeOH and 25 ml. of methylene dichloride was added. After solution was complete, the mixture was poured into dilute aqueous sodium bicarbonate, and the new mixture acidified with dilute hydrochloric acid. The acidic aqueous mixture was extracted with methylene dichloride and the extract dried. Evaporation of the solvent yielded 15.9 g. of a colorless oil comprising methyl cis-(±)-2-benzyloxycarbonylamino-5-hydroxycyclohexanecarboxylate; yield = about 100%. NMR was consistent with the proposed structure.

## Example 5

Preparation of Methyl Cis-(±)-2-benzyloxycarbonylamino-5-oxocyclohexanecarboxylate

A solution was prepared by dissolving 15.8 g. of the alcohol from Example 4 in 400 ml. of acetone. The solution was cooled to about 0°C., and 12 ml. of Jones Reagent added thereto in dropwise fashion over a 30 min. period. TLC indicated complete oxidation of the secondary alcohol to give methyl cis-(±)-2-benzyloxycarbonylamino-5-oxoperhydrobenzoate. The green solid which had formed was separated from the supernatant by decantation, and washed three times with acetone. The supernate and washes were neutralized with aqueous sodium bicarbonate. The neutralized solution was filtered thru CELITE, and the filtrate concentrated. The concentrate was poured into water, and the aqueous mixture extracted with methylene dichloride. The extract was separated and dried, and the solvent removed therefrom. About 15.9 g. of a lightly colored oil (100% yield) comprising methyl cis-(±)-2-benzyloxycarbonylamino-5-oxoperhydrobenzoate were obtained. NMR was consistent with the postulated stucture for the 5-oxo derivative.

## Example 6

Preparation of Methyl cis-(±)-2-benzyloxycarbonylamino-5-ethyleneketalcyclohexylcarboxylate

A reaction mixture, prepared from 51.5 millimoles of methyl cis-(±)-2-benzyloxycarbonylamino-5-oxocyclohexylcarboxylate, 6.4 g. of ethylene glycol, 100 mg. of p-toluenesulfonic acid monohydrate and 250 ml. of benzene, was heated to reflux for about 3 hrs in an apparatus equipped with a Dean-Stark trap to collect water formed as a by-product of the reaction. After the theoretical amount of water had been collected the reaction mixture was allowed to come to room temperature where it was kept overnight. The reaction mixture was

then poured into dilute aqueous sodium bicarbonate, and the bicarbonate layer extracted first with ether and then with methylene dichloride. The extracts were combined and dried. Evaporation of the solvents gave a residue comprising the desired ketal; weight = 17.32 g. The ketal was purified by chromatography over silica using a 2:1 ether/hexane solvent mixture as the eluant. Early fractions gave 14.61 g. (81.5 %) yield of a faintly pink oil comprising purified methyl cis-(±)-2-benzyloxycarbonylamino-5-ethyleneketalcyclohexylcarboxylate.

## Example 7

Isomerization of Methyl Cis-(±)-2-benzyloxycarbonylamino-5-ethyleneketalcyclohexanecarboxylate to the Corresponding Trans-(±) Derivative

A solution of sodium methylate in methanol was prepared by adding 960 mg. of sodium to 200 ml. of methanol. 14.5 g. of methyl cis-(±)-2-benzyloxyamino-5-ethyleneketalperhydrobenzoate were added and the mixture refluxed for 2 3/4 hrs. TLC indicated an equilibrium mixture of the cis and trans racemates. The reaction mixture was poured into dilute hydrochloric acid, and the acidic mixture extracted with methylene dichloride. The organic extract was separated and dried. Evaporation of the solvent gave a residual oil; weight = 14.8 g. The oil was chromatographed over silica using 2:1 ether/hexane as the eluant. Early fractions were shown to be a 5:2 mixture of the trans

and cis isomers. Later fractions were collected and shown by TLC to be nearly pure trans racemate; weight = 8.4 g.

## Example 8

Preparation of Trans-(±)-2-benzyloxyamino-5-ethylene ketal cyclohexylcarbinol

A solution of 8.4 g. of methyl trans-(±)-2-benzyloxycarbonylamino-5-ethyleneketalcyclohexylcarboxylate in 75 ml. of ether was added to a solution of 910 mg. of $LiAlH_4$ in 225 ml. of ether. The reaction mixture was stirred at room temperature for about 10 min., at which time TLC showed no remaining starting material. The reaction was decomposed by the addition, in order, of 0.9 ml. of water, 0.9 ml. of 15% sodium hydroxide and 2.7 ml. of water. The decomposed reaction was filtered to remove inorganic salts. The organic layer was concentrated in vacuo. 4.47 g. of a yellow oil were obtained. Chromatography of the oil over silica using ether as the eluant gave 2.39 (31%) of a colorless oil comprising purified trans-(±)-2-benzyloxycarbonylamino-5-ethyleneketalcyclohexylcarbinol.

## Example 9

Preparation of Trans-(±)-2-benzyloxycarbonylamino-5-ethyleneketalcyclohexylaldehyde

A solution was prepared by dissolving 2.39 g. of the carbinol from Example 8 in 150 ml. of methylene dichloride. Ten grams of pyridinium chlorochromate were added, and the reaction mixture stirred vigorously at room temperature for about 2 hrs. An equal volume of ether was added. The resulting supernatant was separated and flash chromatographed over a silica column. The solid residue from the reaction was rinsed several times with ether and with methylene dichloride, and these rinses also passed through the flash silica column. Concentration of the eluate yielded a residue; total combined residue = about 2.1 g. (88% yield) of the desired trans-(±)-2-benzyloxycarbonylamino-5-ethyleneketalcyclohexylaldehyde.

## Example 10

Preparation of Trans-(±)-2,6-dioxodecahydroquinoline, 6-ethyleneketal

A reaction mixture was prepared by dissolving 1.6 g. of the aldehyde from the Example 9 and 2.5 g. of methyl (triphenylphosphoranylidene)acetate in 40 ml. of benzene. The reaction mixture was heated to reflux temperature for about 19 hrs. and was then concentrated

in vacuo. The residue which showed no aldehyde by TLC, was chromatographed over silica using ether as the eluant. The first product to come off the column was the desired acrylic acid ester, which was a colorless oil; weight = 1.3 g. (70% yield).

A solution was prepared by dissolving 1.30 g. of the above acrylic acid ester in 100 ml. of MeOH. 135 mg. of 5% Pd/C were added, and the mixture hydrogenated by vigourous slurrying under a hydrogen atmosphere. After one-half hour, an additional 250 mg. of catalyst were added, and the mixture slurried for an additional hour under hydrogen. TLC at this point showed no starting material present. The catalyst was separated by filtration, and the filtrate concentrated to give a cloudy oil which solidified on standing. TLC showed a mixture of the decahydroquinoline cyclized product and the uncyclized compound, methyl trans-(±)-3-(2-amino-5-ethyleneketalcyclohexyl)propionate. The product was dissolved in 200 ml. of THF, and the solution heated to reflux temperature for 2 hrs. The solvent was evaporated in vacuo, and the residue chromatographed over silica using THF as the eluant. Fractions 6-13 yeilded 429 mg. of a white solid comprising purified trans-(±)-2,6-dioxo-6-ethyleneketal-decahydroquinoline.

## Example 11

Preparation of Trans-(±)-2,6-dioxodecahydroquinoline

A solution prepared by dissolving 40 mg. of the ketal from Example 10 in 5 ml. of formic acid, was stirred at room temperature for about 30 min. At this time, TLC indicated that the de-ketalization had gone substantially to completion. The reaction mixture was therefore concentrated in vacuo to give 35 mg. of a white solid. The solid was chromatographed over silica using 1:19 $MeOH/CH_2Cl_2$ as the initial eluant followed by 1:9 $MeOH/CH_2Cl_2$ eluant. Later fractions with the second eluant (fractions 6-15) yielded 33 mg. of trans-(±)-2,6-dioxodecahydroquinoline. The compound had the following physical characteristics: Mass spectrum: ions at 167, 111, 110, 97, 84 and 56. Infrared spectrum ($CHCl_3$): 3400, 1716, 1661 $cm^{-1}$.

## Example 12

Preparation of Trans-(±)-6-oxo-4,4a,5,6,7,8,8a,9-octa-hydro-1H(and 2H)-pyrazolo[3,4-g]quinoline

A solution was prepared by dissolving 168 mg. of trans-(±)-2,6-dioxodecahydroquinoline in 10 ml. of warm toluene 0.4 ml. of tris(dimethylamino)methane were added, and the reaction mixture heated to reflux for 2.5 hrs. (A white solid began to appear at 1.5 hrs.) The reaction mixture was cooled to room temperature and then

filtered. 148 mg. of trans-(±)-2,6-dioxo-7-dimethylaminomethylenedecahydroquinoline were collected.

Three milligrams of the above derivative were dissolved in 0.5 ml. of MeOH containing one drop of anhydrous hydrazine added. The reaction mixture was stirred at room temperature for about 2 hrs., and was then dilutied with water. The aqueous phase was extracted with $CH_2Cl_2$ and the extract dried. Removal of the solvent gave 2 mg. of a white solid comprising trans-(±)-6-oxo-4,4a,5,6,7,8,8a,9-octahydro-1H(and 2H)-pyrazolo[3,4-]quinoline formed in the above reaction. NMR on the product was consistent with the proposed structure.

This compound can be reduced with $LiAlH_4$ to yield the tautomeric pair, trans-(±)-4,4a,5,6,7,8,8a,9-octahydro-1H(and 2H)-pyrazolo[3,4-g]quinoline, a useful intermediate to prepare dopamine D-2 agonists by alkylation or allylation of the quinoline ring nitrogen by standard procedures.

## Example 13

Preparation of Trans-(±)-1-n-propyl-2,6-dioxodecahydroquinoline

A solution was prepared by dissolving 390 mg. of trans-(±)-2,6-dioxo-6-ethyleneketaldecahydroquinoline from Example 10 in 15 ml. of THF. This solution was added to a suspension of 150 mg. of sodium hydride in 5 ml. of THF. The reaction mixture was stirred at room

temperature for 1 hr. at which time 2 ml. of DMF and 0.9 ml. of n-propyl iodide were added. This new reaction mixture was stirred at room temperature for 22 hrs. at which time TLC showed that the reaction had gone substantially to completion. The reaction mixture was poured into water and the aqueous mixture extracted with 1:3 isopropanol/chloroform. The extract was separated, and the solvents removed therefrom to give 575 mg. of a yellow oil. Chromatography of the oil over silica using 5:4 THF/pentane as the eluant gave the following results: early fractions (3-5) gave 461 mg. (86% yield) of trans-(±)-1-n-propyl-2,6-dioxo-6-ethyleneketaldecahydroquinoline. The ketal group was removed with formic acid by the procedure of Example 11 to yield trans-(±)-1-n-propyl-2,6-dioxodecahydroquinoline. A 91% yield of the dioxo derivative was obtained. The compound had the following physical characteristics: Mass spectrum: ions at 209, 180, 152, 139, 124, 110.

Substitution of methyl or ethyliodide or of allyl chloride for n-propyliodide in the above example yields, eventually, the corresponding trans-(±)-5-methyl, ethyl or allyl-2,6-dioxodecahydroquinoline.

## Example 14

Preparation of Trans-(±)-5-n-propyl-6-oxo-4,4a,5,6,7,8,8a,9-octahydro-1H(and 2H)-pyrazolo[3,4-g]quinoline

Following the procedure of Example 12, trans-(±)-1-n-propyl-2,6-dioxodecahydroquinoline was

reacted with tris(dimethylamino)methane to yield the corresponding 7-dimethylaminomethylene derivative. This compound was in turn reacted with anhydrous hydrazine to give trans-(±)-5-n-propyl-6-oxo-4,4a,5,6,7,8,8a,9-octahydro-1H(and 2H)-pyrazolo[3,4-g]quinoline in 70% overall yield. Trans-(±)-1-n-propyl-6-oxo-7-dimethylaminomethylenedecahydroquinoline had the following physical characteristics: Mass spectrum; ions at 264, 219, 152, 150, 125, 112, 110, 82. Infrared spectrum: 3465, 1624 $cm^{-1}$.

Reduction of the above 6-oxo derivative with $LiAlH_4$ yields trans-(±)-5-n-propyl-4,4a,5,6,7,8,8a,9-octahydro-1H(and 2H)-pyrazole[3,4-g]quinoline.

While the above synthetic procedure has culminated in the production of trans-(±)-5-n-propyl-6-oxo-4,4a,5,6,7,8,8a,9-octahydro-1H(and 2H)-pyrazolo-[3,4-g]quinoline, the intermediate trans-(±)-1-n-propyl(or methyl, ethyl or allyl)-2,6-dioxodecahydroquinoline from Example 13 or the corresponding 1-unsubstituted derivative from Example 11 can be reacted with bromine to yield a 7-bromo derivative and that intermediate reacted with an isothiourea of the formula

$$\begin{array}{c} \quad NH \\ \quad \| \\ HS-C-NR^1R^2 \end{array}$$

wherein each of $R^1$ and $R^2$ is individually hydrogen, methyl, ethyl or n-propyl.

The product of this reaction is a racemic trans-(±)-7-amino($NR^1R^2$)-5-permissibly substituted-6-oxo-4,4a,5,6,7,8,8a,9-octahydro thiazolo[4,5-g]quinoline,

composed of the enantiomers XXXa and XXXb below or with an enantiomeric starting material to yield either XXXa or XXXb.

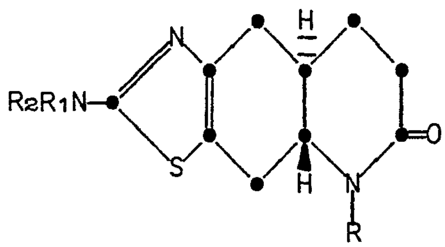

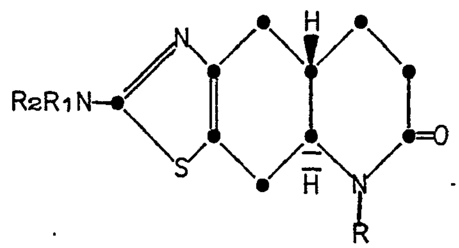

XXXa XXXb

wherein $R^1$ and $R^2$ have their previous meaning and R is H, methyl, ethyl, n-propyl or allyl.

The 6-oxo group can then be reduced with a chemical reducing agent such as $LiAlH_4$ to yield the racemic or enantiomeric dopamine D-2 agonist disclosed in Titus and Kornfeld (E.P. Patent Application Serial No. 85302852.0) the trans-(±)-2-amino-5-permissibly substituted octahydrothiazolo[4,5-g]quinoline XXXIa (4aR,8aR) and XXXIb (4aS,8aS).

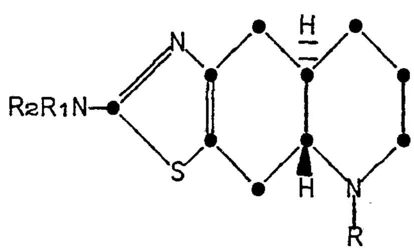

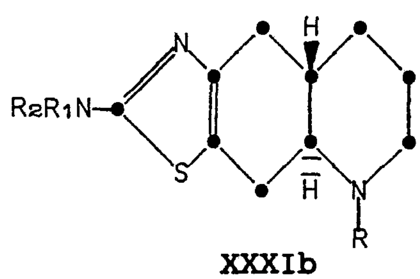

XXXIa XXXIb

In XXXIa and XXXIb, if R is H, the quinoline nitrogen must be alkylated or allylated to give a dopamine agonist (D-1 or D-2). Since the cyclization to yield an thiazole ring is carried out on a 6-bromo-5-oxodecahydroquinoline, and since bromination might also react with the double bond of a 1-allyl derivative, to prepare such allyl compounds, it is preferred to brominate the trans-(±)-1-unsubstituted 2,6-dioxodecahydroquinoline, cyclize the bromo derivative with isothiourea to yield a 6-oxothiazolo[4,5-g]quinoline, remove the 6-oxo group with $LiAlH_4$ and then allylate at N-5 to give the N-allyl dopamine agonists. Alternatively, the order of the $LiAlH_4$ reduction and N-5 alkylation steps can be reversed.

Similarly, the reaction of a trans-(±)-1-permissibly-substituted-2,6-dioxo-7-bromodecahydroquinoline or a 4aR,8aR or a 4aS,8aS enantiomer thereof with an isourea of the formula

$$HO-\overset{\overset{NH}{\|}}{C}-NR^1R^2$$

yields a racemic trans-(±)-2-amino-5-permissibly-substituted-6-oxo-4,4a,5,6,7,8,8a,9-octahydro[4,5-g]-quinoline, structures XXXIIa (4aR,8aR) and XXXIIb (4aS,8aS) or the individual enantiomers thereof.

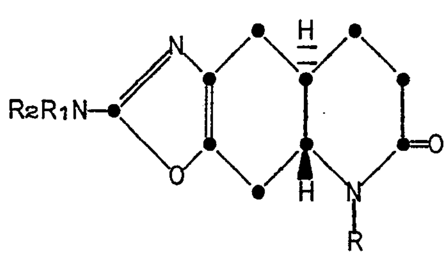

and

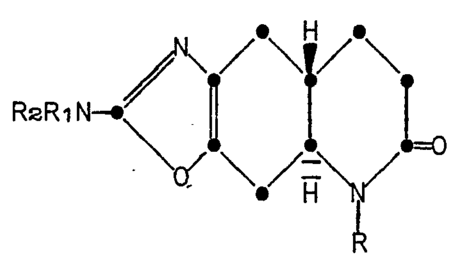

XXXIIa XXXIIb

Reduction of the racemate or either enantiomer with a chemical reducing agent such as $LiAlH_4$ yields the 2-amino-5-substituted octahydrooxazolo[4,5-g]quinolines of our copending application of even date, agent's reference X-6599A of structures XXXIIIa (4aR,8aR or XXXIIIb (4aS,8aS)

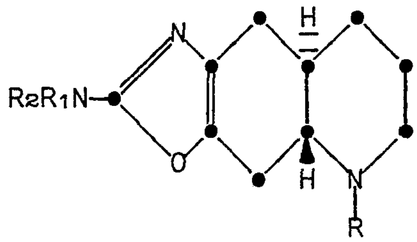

XXXIIIa

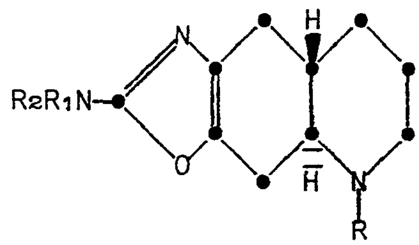

XXXIIIb

wherein R is H, $C_{1-3}$ straight-chain alkyl or allyl.

As with the thiazoles, preparation of a compound according to XXXIIIa or XXXIIIb where R is allyl, is better carried out by allylating a 2-amino-6-oxo-octahydro-oxazolo[4,5-g]quinoline (XXXIIa and XXXIIb) and then reducing the N-allyl lactam or by allylating the final oxazolo[4,5-g]quinoline (XXXIIIa and XXXIIIb when R is H).

Reaction of the racemate XXIV (from Synthetic Route 1) or an enantiomer thereof with guanidine or a substituted guanidine ($R^2R^1\text{-N-}\overset{\overset{\text{NH}}{\|}}{\text{C}}\text{-NH}_2$) yields a 7-oxo-6-permissibly-substituted octahydropyrimido[4,5-g]quinoline of the formulas, XXXVa (5aR,9aR) and XXXVb (5aS,9aS)

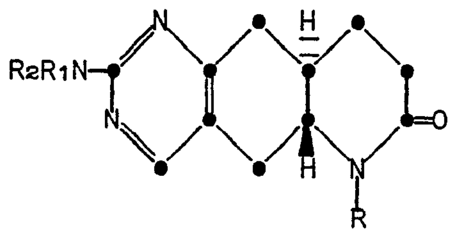

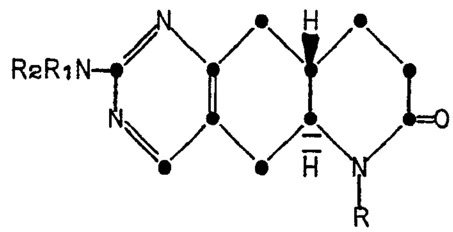

XXXVa XXXVb

where R is H, $C_{1-3}$ straight-chain alkyl or allyl and $R^1$ and $R^2$ have their previous meaning. Reduction of the lactam with a chemical reducing agent such as $LiAlH_4$, optionally followed by alkylation or allylation where R is H, yields the powerful dopamine D-2 agonists, the racemate and the 5aR,9aR enantiomer, XXXVIa and the D-1 agonist, the enantiomer XXXVIb (In the D-1 and D-2 agonists, R is not H) described in U.K. Patent Application No. 8421160.

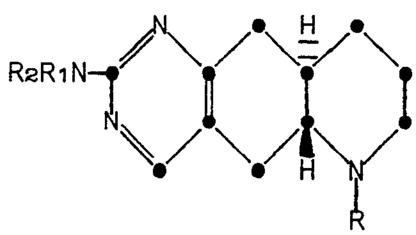

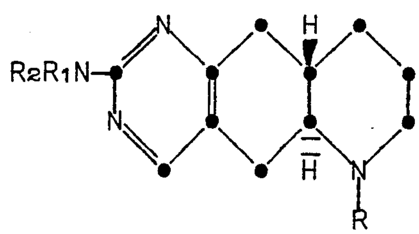

XXXVIa XXXVIb

## Example 15

Preparation of trans-(±)-5-n-propyl-4,4a,5,6,7,8,8a,9-octahydro-1H(and 2H)-pyrazolo[3,4-g]quinoline

trans-(±)-5-n-Propyl-6-oxo-4,4a,5,6,7,8,8a,9-octahydro-1H(and 2H)-pyrazolo[3,4-g]quinoline (17 mg.) in THF (2.5 ml.) was added to a suspension of lithium aluminum hydride (19 mg) in THF (5.0 ml). The reaction mixture was stirred at room temperature for three hours and quenched by the addition of two drops of 15% NaOH solution. The reaction mixture was then stirred vigorously for 18 hours, filtered and concentrated to give 13 mg. of a colorless oil. This material was chromatographed on a flash silica gel column using 15% methanol in $CH_2Cl_2$ and ammonium hydroxide (trace) as solvent.

The chromatographed material was identical in all essential respects (NMR, TLC etc) with authentic quinpirole.

## Example 16

Urine from monkeys given a 2 mg/kg. (or 20 mg/kg.) nasogastric dose of $^{14}C$-4aR,8aR-5-n-propyl-4,4a,5,6,7,8,8a,9-octahydro-1H(and 2H)-pyrazolo[3,4-g]-quinoline was applied to a Diaion HP-20 column. The column was washed with water until radioactivity was detected in the eluate. The radioactivity (ave. 94%) was then eluted with methanol. Radioactivity in the residue that resulted upon evaporation of the methanolic fraction was then selectively dissolved in a minimal volume of various organic solvents (methanol, ethanol,

and ethanol, ethyl acetate (2:1)). This treatment caused precipitation of endogenous substances and allowed the radioactivity to be concentrated in a small volume of methanol suitable for thin-layer chromatography on silica gel plates [chloroform/methanol/15N ammonium hydroxide (50:45:5)]. Autoradiography of the developed TLC plates revealed five broad bands. Regions corresponding to the bands were scraped from the plates, the radioactive metabolites eluted from the scrapings with methanol, and the eluates evaporated to dryness under reduced pressure.

The radioactive material eluted from the TLC band ($R_f$ = 0.58-0.85) was dissolved in deionized water and the pH adjusted to ca. 12 with .01 N aqueous NaOH. The desired radioactive metabolites were extracted into ethyl acetate. Evaporation of the combined ethyl acetate extracts yielded a residue which was dissolved in .001N HCl.

The acidic aqueous layer was extracted with ethyl acetate, and the ethyl acetate extracts discarded. The pH of the aqueous layer was then adjusted to ca. 12 with 5N NaOH and the radioactive metabolite was extracted into ethyl acetate. The ethyl acetate extracts were evaporated to dryness under reduced pressure, and the resulting residue was dissolved in deionized water. Injection of this aqueous layer into the following HPLC solvent system: 100% water-5 min; 0-100% methanol-37.5 min at 2 ml/min flow rate (using an Alltech, C8, 10μ column). This procedure gave the tautomeric pair, 4aR,8aR-6-oxo-4,4a,5,6,7,8,8a,9-octahydro-1H(and 2H)-pyrazolo[3,4-g]quinoline, in substantially pure form. The product was further purified by HPLC using the same solvent system with a Whatman Partisil 5, ODS-3, RAC column.

The compound thus purified had the following physical characteristics:

Mass spectrum (Field desorption); molecular ion at 191; (High resolution electron impact) 191 (molecular ion), 119,94

Infrared spectrum: ($CHCl_3$) 1660 $cm^{-1}$ (lactam carbonyl)

NMR ($CDCl_3$) $\delta$ at 7.34 (H3), ~2.5 (H4ax), 2.89 (H4eq), 3.41 (H4a), ~2.5 (H7ax), ~2.6 (H7eq), 1.70 (H8ax), 2.05 (H8eq), 1.90 (H8a), ~2.4 (H9ax), 2.99 (H9eq).

Using the same TLC fraction as above ($R_f$ = 0.58-0.85), the radioactive material was dissolved in .01 N aqueous NaOH and the radioactive material was extracted into ethyl acetate. The ethyl acetate extract was washed with .001 N HCl. The pH of the acidic aqueous layer was adjusted to neutrality using 1N aqueous NaOH. Injection of the aqueous extract onto the following HPLC system: 100% water 5 min; 0-100% methanol 37.5 min at a 2 ml/min flow rate (Whatman Partisil 5, ODS-3, RAC column). This procedure gave the tautomeric pair 4aR,8aR-5-n-propyl-6-oxo-4,4a,5,6,7,8,8a,9-octa-hydro-1H(and 2H)-pyrazolo[3,4-g]quinoline. The product was further purified by HPLC using with the following solvent system but the same column:100% water-7 min; 0-60% methanol-20 min at a 2 ml/min flow rate. The

compound thus purified had the following physical characteristics:

Mass Spectrum (Field desorption); molecular ion at 233; (High resolution electron impact) 233 (molecular ion), 204,190,176,119,94.

Infrared spectrum ($CHCl_3$): 1626 $cm^{-1}$ (lactam carbonyl)

NMR ($CDCl_3$): δ at 7.37 (H3), 3.21 (H4eq), 2.39 (H4ax), 3.34 (H4a), 2.56 (7eq), 2.48 (H7ax), 1.94 (H8eq), 1.63 (H8ax), 1.94 (H8a), 2.97 (H9eq), 2.48 (H9ax), 3.75 and 3.23 (H10), 1.71 and 1.50 (H11), 0.92 (H12).

## Example 17

Preparation of trans-(±)-2-amino-7-oxo-5,5a,6,7,8,9,9a,10-octahydropyrimido[4,5-g]quinoline

trans-(±)-2,6-Dioxo-7-dimethylaminomethylene-decahydroquinoline (28 mg) was dissolved in ethanol (6 ml) and guanidine carbonate (20 mg) added. This mixture was heated to reflux for 1½ hours whereupon a precipitate formed. The reaction mixture was then allowed to cool to room temperature and filtered to yield the title compound as a white powder (22 mg).

Mass spectrum m/e 218 (100), 219 (30).

## Example 18

Preparation of trans-(±)-2-amino-5,5a,6,7,8,9,9a,10-octahydropyrimido[4,5-g]quinoline

trans-(±)-2-Amino-7-oxo-5,5a,6,7,8,9,9a,10-octahydropyrimido[4,5-g]quinoline (10 mg) was suspended in tetrahydrofuran (7 ml) and heated to reflux. Lithium aluminum hydride (9 mg) was added to the suspension and the reaction mixture refluxed for 3 hours. The reaction was quenched by the addition of two drops of 15% NaOH solution and the reaction mixture was then stirred vigorously for 17 hours. The reaction mixture was filtered and the solid from the filtration treated with boiling 1:1 methanol/chloroform solution. This mixture was filtered and the combined filtrates concentrated to give crude title product as a white solid (8 mg).

A portion of the crude product was chromatographed on a flash silica gel column using methanol containing a trace of $NH_4OH$ as eluent. Fractions containing the desired pure material were combined to give a white solid (1 mg).

m/e 204 (100), 205 (15).

## Example 19

Using procedures similar to those described above in Examples 17 and 18, trans-2-amino-6-n-propyl-5,5a,6,7,8,9,9a,10-octahydropyrimido[4,5-g]quinoline can be prepared.

## CLAIMS

1. A process for preparing a tricyclic quinoline derivative of formula:

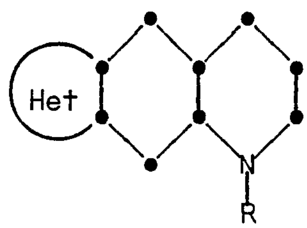

(I)

where the

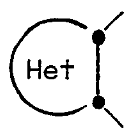

group represents:

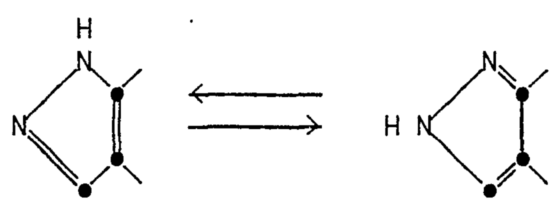

,

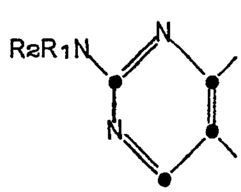

,

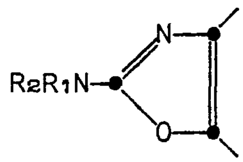

or

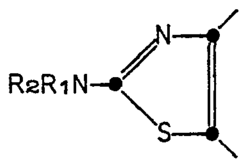

where $R_1$ and $R_2$ are independently hydrogen or $C_{1-3}$ alkyl; and R is hydrogen, $C_{1-3}$ straight chain alkyl or allyl; or a pharmaceutically-acceptable salt thereof; which process comprises reducing a lactam of formula:

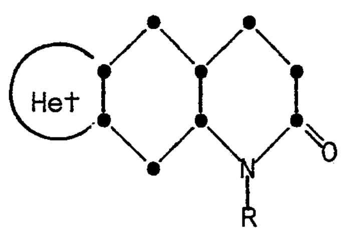

(II)

the ring-fusion of the non-aromatic rings being trans.

2. A process according to claim 1 wherein

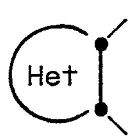

represents

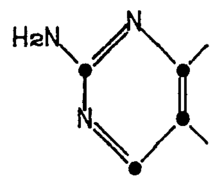

and wherein R is hydrogen or $C_{1-3}$ straight chain alkyl.

3. A process according to claim 1 or 2 wherein the reduction is carried out with lithium aluminum hydride.

4. A lactam of formula:

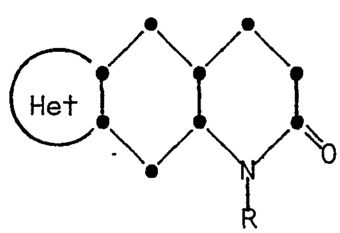

(II)

where the

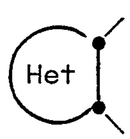

group represents:

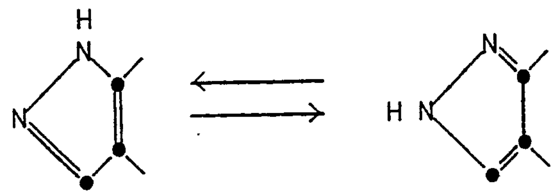

,

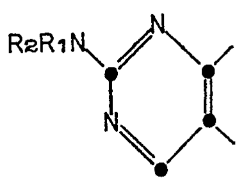

,

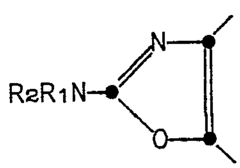

or

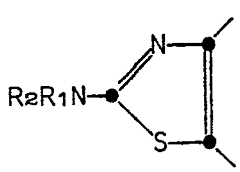

where $R_1$ and $R_2$ are independently hydrogen or $C_{1-3}$ alkyl; and R is hydrogen, $C_{1-3}$ straight chain alkyl or allyl; and the ring-fusion of the non-aromatic rings is <u>trans</u>.

5. A compound of formula:

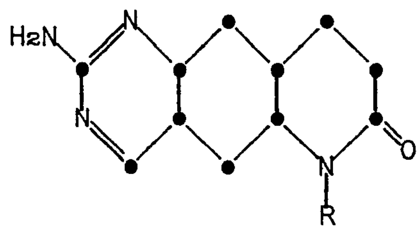

wherein R is hydrogen or $C_{1-3}$ straight chain alkyl and wherein the ring-fusion of the non-aromatic rings is <u>trans</u>.

## CLAIMS

1. A process for preparing a tricyclic quinoline derivative of formula:

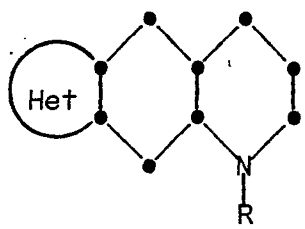

(I)

where the

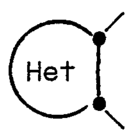

group represents:

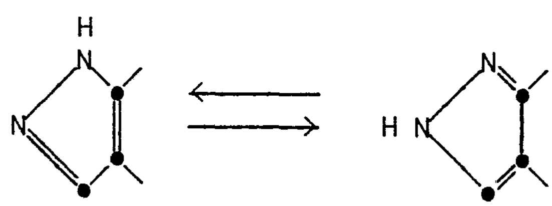

,

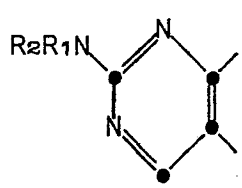

,

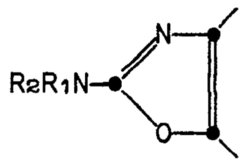

or

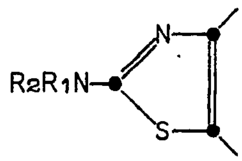

where $R_1$ and $R_2$ are independently hydrogen or $C_{1-3}$ alkyl; and R is hydrogen, $C_{1-3}$ straight chain alkyl or allyl; or a pharmaceutically-acceptable salt thereof; which process comprises reducing a lactam of formula:

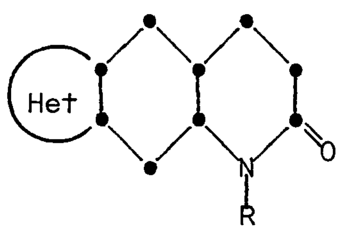

(II)

the ring-fusion of the non-aromatic rings being trans.

2. A process according to claim 1 wherein

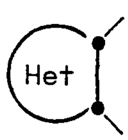

represents

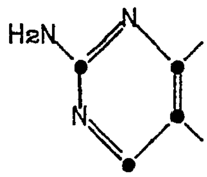

and R is hydrogen or $C_{1-3}$ straight chain alkyl.

3. A process according to claim 1 or 2 wherein the reduction is carried out with lithium aluminum hydride.